# EUROPEAN PATENT APPLICATION

(11) **EP 3 370 176 A1**
(43) Date of publication of application: **05.09.2018**
(21) Application number: 17159200.9
(22) Date of filing: 03.03.2017
(51) Int. Cl.: G06F 19/00, A61B 5/11

(54) **PROVIDING ACTIVITY INFORMATION IN RELATION TO INTERACTIVE USER BEHAVIOR**

(71) Applicant: vitaliberty GmbH, 68165 Mannheim (DE)
(72) Inventor: GRABOW, Janina, 68163 Mannheim (DE); KAISER, Daniel, 55232 Alzey (DE); KARLE, Alexander, 68163 Mannheim (DE); STERNSDORF, Björn, 76137 Karlsruhe (DE); SCHWEIZER, Marvin Connor, 76287 Rheinstetten (DE)
(74) Representative: Cohausz & Florack

(57) **Abstract**

A method is disclosed comprising: causing, at least in part, providing or providing an activity information indicative of an activity to be performed by a user, obtaining user activity information, wherein the user activity information is indicative of at least one inclusion criterion, at least one exclusion criterion, at least one controlling criterion, or a combination thereof, wherein at least a first part of the user activity information is gathered by a sensor and at least a second part of the user activity information is received by user input, determining, using a processor of the at least one apparatus, an adaptation of the activity information based on the user activity information, outputting the activity information. It is further disclosed an according apparatus, computer program and system.

## Description

### FIELD

The following disclosure relates to systems, apparatuses, and methods for a server providing an activity information or a terminal receiving an activity information for providing a service to a user to monitor, support the fitness/healthcare of the user.

### BACKGROUND

Exercise and fitness have become increasingly popular not only for athletes, but also for users wanting to obtain or maintain a certain level of fitness.

Various types of terminals are known for use in fitness for instance to provide performance data, and recording their activities.

Usually, activities, e.g. fitness related exercises, are carefully chosen by the users or are a strict instruction by a predefined program (e.g. fitness related training program). Most programs pursue a change in physical or mental conditions, but are not able to react programmatically if such change happens. Often times, users have to adjust their program by themselves. Further, the predefined program may not suit the athlete properly. This may often times be very time consuming and it may be hard for the user to reach a certain goal. Furthermore predefined programs usually do not cover hick-ups in the users individual routine as they are solely focusing on the programmatic "happy flow" (100% progress by the user) which is for the vast majority of users not the case as they struggle with real life problems and are stuck in existing habits. Thus, quitters are very common.

Based on this prior art, it is object of the invention to provide a technical solution to enhance the user experience and making it as easy as possible for a user to acquire a certain goal or behavioral change, e.g. related to his fitness.

### SUMMARY

Small activities (e.g. exercises) resulting often times in the same results as time consuming limited activities chosen by a user. Further, small activities can be easily implemented into the daily routines since they only require minimal effort and time.

It is thus, inter alia, an object of the present invention to provide a solution in which an activity information indicative of an activity (e.g. exercise) can be easily implemented into daily routines of the user, and can be provided to the user.

According to a first exemplary aspect a method, e.g. performed by at least one apparatus, is disclosed, the method comprising: causing, at least in part, providing or providing an activity information indicative of an activity to be performed by a user; obtaining user activity information, wherein the user activity information is indicative of at least one inclusion criterion, or at least one exclusion criterion, or at least one controlling criterion, or a combination thereof, wherein at least a first part of the user activity information is gathered (e.g. by a sensor of a terminal, or by a tracking of the terminal, e.g. of the user) and at least a second part of the user activity information is received by user input; determining, using a processor of the at least one apparatus, an adaptation of the activity information based on the user activity information; outputting the activity information.

This method may for instance be performed and/or controlled by an apparatus, e.g. a server. Alternatively, this method may be performed and/or controlled by more than one apparatus, e.g. a server cloud comprising at least two servers. This method may for instance be performed and/or controlled by a terminal, e.g. a smartphone, tablet, wearable.

According to a further exemplary aspect, a computer program is disclosed, the computer program when executed by a processor causing an apparatus (e.g. a server) to perform and/or control the actions of the method according to the first exemplary aspect of the invention.

The computer program may be stored on computer-readable storage medium, in particular a tangible and/or non-transitory medium. The computer readable storage medium could for example be a disk or a memory or the like. The computer program could be stored in the computer-readable storage medium in the form of instructions encoding the computer-readable storage medium. The computer-readable storage medium may be intended for taking part in the operation of a device, like an internal or external memory (e.g. a Read-Only Memory (ROM)) or hard disk of a computer, or be intended for distribution of the program, like an optical disc or a cloud storage accessible e.g. via the internet.

According to a further exemplary aspect of the invention, an apparatus is disclosed, configured to perform and/or control or comprising respective means for performing and/or controlling the method according to the first exemplary aspect of the invention.

The means of the apparatus can be implemented in hardware and/or software. They may comprise for instance at least one processor for executing computer program code for performing the required functions, at least one memory storing the program code, or both. Alternatively, they could comprise for instance circuitry that is designed to implement the required functions, for instance implemented in a chipset or a chip, like an integrated circuit. In general, the means may comprise for instance one or more processing means or processors.

According to a further exemplary aspect of the invention, an apparatus is disclosed, comprising at least one processor and at least one memory including computer program code, the at least one memory and the computer program code configured to, with at least one processor, cause an apparatus (e.g. the apparatus) at least to perform and/or control the method according to the first exemplary aspect of the invention.

The above-disclosed apparatus according to any aspect of the invention may be a module or a component for a device, for example a chip. Alternatively, the disclosed apparatus according to any aspect of the invention may be a device, for instance a server or a server cloud or a terminal. The disclosed apparatus according to any aspect of the invention may comprise only the disclosed components (e.g. means, processor, memory) or may further comprise one or more additional components.

According to a further exemplary aspect, a system is disclosed, comprising: one or more apparatuses according to any aspect of the invention as disclosed above, in particular at least one server and a terminal, which are adapted to at least perform and/or control the method according to any aspect of the invention.

In the following, exemplary features and exemplary embodiments of all aspects will be described in further detail.

The activity information is indicative of an activity to be performed by a user. The activity information may for instance be indicative of a topic associated with user activity information, e.g. fitness, nutrition, psychology, mental or issues such as hypertension to name but a few examples. The activity to be performed by the user may for instance be a fitness related exercise or whole workout, a request to the user to enter and/or input information, e.g. actual weight of the user, or a recommendation to the user for better time management, or a combination thereof, to name but a few examples.

For instance, the activity information may be associated to an activity and/or exercise, which may be proposed to the user to be performed by the user. The activity information may for instance be presented to the user, e.g. by displaying the activity information. The activity information may for instance comprise at least one instruction how a certain activity and/or exercise can be performed.

The user activity information may for instance stem from the terminal. It may for instance be received from the user, e.g. by user input of the user activity information. The user activity information may for instance be received from another entity, e.g. from a server or computer.

The user activity information may for instance comprise at least one of the following: (i) one or more inclusion criterion; (ii) one or more exclusion criterion; (iii) one or more controlling criterion; (iv) one or more influencing criterion; (v) one or more user behavior criterion; or a combination thereof.

Inclusion criterion may for instance be indicative of one or more parameters of age, height, body mass index (BMI), gender, fitness level, goal and/or motivator or barrier of the user. For instance, a user of a lower age and higher fitness level may for instance be able to perform more difficult exercises and/or activities compared to a user of higher age and lower fitness.

Exclusion criterion may for instance be indicative of a certain barrier of the user. The barrier may prevent the user from performing a certain activity (e.g. exercise). The barrier of the user may for instance represent a physical constraint of the user, and/or an available time frame of the user, in which time frame the user is prevented (due to personal and/or professional objectives) to perform an activity (e.g. exercise), which is caused to be provided to the user by an activity information. Alternatively or additionally, another exclusion criterion may for instance be user feedback, e.g. provided (e.g. entered into a terminal of the user) by the user. For instance, the user may for instance provide user input indicative of the user not liking a certain activity of the provided or caused providing activity information, the certain activity may for instance be determined as an exclusion criterion resulting in not outputting the certain activity of the activity information to the user (again in a further execution of the present invention).

Controlling criterion may for instance be indicative of a difficulty, intensity, extension, pause, frequency, or a combination thereof of an activity (e.g. exercise) provided to the user.

The difficulty may for instance be separated in one or more grades, e.g. grades 1 to 5, wherein grade 1 relates to a very low difficulty of an activity (e.g. exercise), and grade 5 relates to a very intense difficulty of an activity (e.g. exercise) provided to the user.

The intensity may for instance be separated in one or more grades, e.g. grades 1 to 5, wherein grade 1 relates to a very low intensity of an activity (e.g. exercise), and grade 5 relates to a very intense intensity of an activity (e.g. exercise) provided to the user.

The extension may for instance be separated in one or more grades, e.g. grades 1 to 5, wherein grade 1 relates to a very low extension of an activity (e.g. exercise), and grade 5 relates to a very intense extension of an activity (e.g. exercise) provided to the user.

The pause may for instance be separated in one or more grades, e.g. grades 1 to 5, wherein grade 1 relates to a pause until a total regeneration of the user after performing an activity (e.g. exercise), and grade 5 relates to a no pause for the user after performing an activity (e.g. exercise) provided to the user.

The frequency may for instance be separated in one or more grades, e.g. grades 1 to 5, wherein grade 1 relates to performing training and/or routine by the user once per week, grade 2 relates to performing training and/or routine by the user up to twice per week, grade 3 relates to performing training and/or routine by the user up to three times per week, grade 4 relates to performing training and/or routine by the user daily, and grade 5 relates to performing training and/or routine by the user multiple times daily.

The first part of the user activity information is obtained (e.g. gathered) by a sensor, e.g. of a terminal of the user. The sensor of the terminal may for instance be an optical sensor, e.g. a camera, gathering picture information being representing the user performing the activity (e.g. exercise) of the user activity information. Additionally or alternatively, the sensor of the terminal may for instance be an inertial measurement unit sensor (e.g. accelerometer, gyroscope, magnetometer, barometer); biopotential sensor (e.g. heart rate monitoring sensor, ECG, EEG, EMG monitoring); stretch sensor, pressure sensor, impact sensor (e.g. resistive force sensor, capacitive pressure sensor); chemical sensor, temperature sensor, acoustical sensor. Additionally or alternatively, the first part of the user activity information may be obtained by a tracking, e.g. performed and/or controlled by a terminal of the user. The tracking may for instance be a time-based tracking, e.g. an activity of an activity information takes a certain amount of time to be performed. In case the activity of said activity information was outputted for the certain amount of time (e.g. and the outputting of the activity information was not aborted), the activity of the activity information is tracked to be completed successfully.

The second part of the user activity information may for instance be obtained (e.g. entered) by user input. For instance, a questionnaire may be provided to the user, wherein in particular inclusion criteria and/or exclusion criteria comprised by the user activity information can be obtained. Alternatively or additionally, the second part of the user activity information may for instance be a feedback information provided (e.g. inputted or entered) by the user. For instance, the user may confirm that an activity of the activity information was performed by him/her. Entering the second part of the user activity information may for instance be performed and/or controlled by using an input device, e.g. a keyboard coupleable or comprised by the terminal of the user.

User behavior criterion may for instance be indicative of an amount of times the user has performed an activity (e.g. exercise) provided to the user. Further, user behavior criterion may for instance be indicative of an analysis of interest of the user in activity information provided by e.g. analyzing whether the user has chosen to perform an activity (e.g. exercise) provided by the activity information to name but an example.

A set of measures may for instance comprise one or more activity information. The set of measures may be predefined and may comprise one or more activity information related to exercises and/or activities being alternatives to each other, or being classified as having the same or nearly the same of at least one of the following: (i) one or more inclusion criterion; (ii) one or more exclusion criterion; (iii) one or more controlling criterion; (iv) one or more influencing criterion; (v) one or more user behavior criterion; or a combination thereof.

One or more activity information may for instance be comprised by a set of measures. In particular, the one or more activity information of a set of measures may be representing a certain training workout. A training workout activity (e.g. exercise) may for instance be indicative of at least one of the following information: (i) daily trainings and/or daily routines; (ii) weekly trainings and/or routines; (iii) warm-up trainings and/or routines; (iv) main workout trainings and/or routines; (v) recipes; (vi) motivational activities; (vii) instructions with respect to trainings and/or routines, or a combination thereof.

Instructions may for instance comprise components of the trainings and/or routines, e.g. frequency, intensity, calorie consumption to name but a few examples of the activity (e.g. exercise) to be performed by the user.

Providing the activity information may for instance be performed by displaying the activity information on a display of a terminal of the user.

Causing, at least in part, e.g. by using the processor, providing an activity information indicative of an activity and/or exercise to be performed by a user may for instance comprise causing, at least in part, e.g. performed and/or controlled by the terminal, to gather information about a tracking (e.g. monitoring) of the activity (e.g. exercise) performed by the user. The gathering of information about a tracking of the activity (e.g. exercise) may for instance be performed and/or controlled by a sensor of the terminal. For instance the sensor can be one of the aforementioned sensors, e.g. optical sensor, inertial measurement unit sensor, etc.

The determining, using a processor of the at least one apparatus, of the adaptation of the activity information based on the user activity information may for instance be performed and/or controlled by the processor of the at least one apparatus, e.g. the terminal of the user. The adaptation may alternatively be performed and/or controlled by an entity that is different from the terminal of the user. The determining is for instance performed by a server or server cloud that have the respective activity information and/or user activity information.

The outputted activity information may for instance be the adapted activity information. The outputting of the activity information may for instance be by providing, or causing providing of the activity information to a terminal, in particular the terminal of the user. The adapted, in particular updated activity information is output, e.g. to the terminal, or to another apparatus that transfers the activity information to the terminal.

The apparatus may for instance comprise or be connectable to a display for displaying the activity information to the user. The apparatus may for instance comprise or be connectable to one or more sensors for determining the apparatus position, such as for instance a Global Navigation Satellite System (GNSS) receiver, e.g. in the form of a Global Positioning System (GPS) receiver.

Example embodiments thus make it possible to determine (e.g. at a server or server cloud) an adaptation of the activity information based on user activity information, wherein the user activity information is obtained adaptively based on the performance of the user of performed exercises and/or activities and on user input. The activity information is (interactively) adapted in order to increase the user-friendliness of exercise- and/or activity-based services, in particular making it as easy as possible for a user to acquire a certain behavioral change related to his favored topic, e.g. fitness.

According to an exemplary embodiment of all aspects, the method further comprises:
- determining a contextual information based, at least in part, on the activity information, wherein the contextual information is indicative of further information associated with the activity information.

Additionally, the method may for instance further comprise outputting the contextual information. The outputting of the contextual information may for instance be by providing, or causing providing of the contextual information to a terminal, in particular the terminal of the user e.g. from the at least one apparatus.

The contextual information may for instance be output, e.g. to the terminal, or to another apparatus that transfers the activity information to the terminal.

The user may for instance react to such contextual information, e.g. presented to the user by the outputting of the contextual information. The contextual information may for instance be determined by a processor, e.g. of the at least one apparatus.

The further information, e.g. activity formats, may for instance be one or more quizzes, recipes, diary entries, documentations, inputted parameters associated with at least one feeling of the user, in particular at least one feeling of the user after performing activity information, (e.g. professional) articles, tips, motivational quotes, or a combination thereof associated with the user activity information. The parameters may for instance be inputted by the user by entering the parameter, e.g. into a terminal of the user. For instance, the user may enter a value between a predefined range (e.g. between a range of 1 to 10, 1 indicating the user feeling bad after a certain activity of the activity information the user has performed, and 10 indicating the user feeling good) indicating whether or not or how good the user feels after an activity of an activity information was performed.

According to an exemplary embodiment of all aspects, movement information, e.g. from an electronic device, e.g. the at least one apparatus or another terminal (e.g. smartphone, wearable) or the terminal of the user, are obtained, wherein the gathered movement information is indicative of a movement caused by the activity performed by the user. For instance, the movement information can be gathered by an electronic device (e.g. terminal or wearable) comprising at least one sensor, as disclosed above. The movement information may for instance comprise information gathered by the at least one sensor and is gathered during the performance of an activity (e.g. exercise) by the user. For instance, the electronic device can be a wearable, which the user may wear at the wrist. During the performance of an activity, e.g. exercise, the wrist of the user should move in a certain kind of movement. At least one sensor of the electronic device gathers technical data of the movement by the user. Alternatively or additionally, the movement information may for instance be obtained (e.g. received or gathered) by a (e.g. automatic) tracking of the activity information provided, e.g. by a terminal, in particular the terminal of the user, or by another entity (e.g. a wearable of the user). For instance, after the activity information is provided, it may for instance be tracked whether or not the provided activity information is received by the user completely (e.g. the user has not aborted the provision (e.g. displaying) of the activity information or the user has not exited an application providing the activity information to the user). Alternatively or additionally, the movement information may for instance be obtained (e.g. utilized) from an application programming interface (API). The API may for instance utilize the access to a further electronic device or to a further application, which can provide the movement information.

A first feedback information is determined, e.g. by using the processor of the at least one apparatus, wherein the first feedback information is indicative of whether the activity (e.g. exercise) is performed correctly. The determining may for instance be performed based on a comparison of the gathered movement information to at least one reference value information indicative of the activity (e.g. exercise) being performed correctly. An activity (e.g. exercise) performed correctly can be understood as the particular information gathered for instance by at least one sensor of the electronic device being moved in the intended kind of way. For instance, in case the electronic with comprising the sensor can be worn at a wrist by a user, the information gathered by the at least one sensor of the electronic device represent the movement corresponding to the activity (e.g. exercise) in the intended kind of way. In an exemplary embodiment reference value information may for instance be stored in a database, wherein the information may for instance be gathered in advance by gathering the respective movement information when the activity (e.g. exercise) is performed by a reference person. In an exemplary embodiment an amount of divergence between the gathered movement information and the reference value information may for instance be determined, e.g. by a respective calculation.

Additionally or alternatively, the determining of a first feedback information may for instance be performed by a given first feedback information by the user (e.g. entered by the user) during or after an activity indicative of whether the activity was recommended correctly by the system (e.g. intensity of a workout or level of difficulty of an exercise to name but a few examples).

The first feedback information may for instance be used to cause the electronic device to provide at least one of an optical, an acoustical or a haptic feedback to the user. Based on the first feedback information, the user may for instance be able to enhance the performance of the activity (e.g. exercise) of the activity information.

The first feedback information is caused providing or provided, e.g. to the user.

According to an exemplary embodiment of all aspects, the method further comprises: determining, e.g. by the processor of the at least one apparatus, a second feedback information indicative of an effort of the user to perform the activity (e.g. exercise) based on the comparison to another reference value information. For instance, it may be evaluated whether the activity was too easy or too hard or 'just right' to be performed by the user. The activity performed 'just right' may be equal to the activity being performed correctly, as described in this specification above. The other reference value information used for the comparison may for instance be stored in a database. The gathering of the other reference value information may for instance be performed in advance by gathering said information from at least one reference person.

The second feedback information is caused providing or provided, e.g. to the user.

According to an exemplary embodiment of all aspects, the determining of the adaption is caused, at least in part, based at least on one or more of the following parameters:
(i) one or more movement information;
(ii) one or more location information representative to a current surrounding of the user and/or the position of the user;
(iii) one or more weather information representative to current weather conditions influencing an activity (e.g. exercise) being possible to be performed;
(iv) one or more user input with respect to (a) at least one inclusion criterion; (b) at least one exclusion criterion; (c) at least one controlling criterion; (d) at least one influencing criterion; (e) at least one user behavior criterion; or a combination thereof.

For instance, a certain activity (e.g. exercise) may be linked to at least one of the parameters. Thus, the activity information may for instance be determined based on the parameters. Each parameter may influence the determining of the adaptation of the activity information. For instance, when a user input is received, which may change at least one of the parameters, the determining of the adaption may be performed.

According to an exemplary embodiment of all aspects, the determining of the adaptation is performed and/or controlled in predetermined time intervals. For instance, the determining may for instance be performed and/or controlled in predetermined time intervals of e.g. every 24 hours, 8 hours, after each performed activity (e.g. exercise) to name but a few examples. The determining of the adaption may for instance be performed and/or controlled after receiving user input indicating a request for performing and/or controlling the determining of the adaption.

According to an exemplary embodiment of all aspects, the method further comprises: determining a user behavior information based, at least in part, on user input, wherein the user behavior information is indicative of an interest of the user in the activity information provided to the user, wherein the user behavior information is determined based on at least one of the following:
(i) one or more representatives of indicator information, wherein the indicator information is obtained by user input;
(ii) one or more representatives of information being indicative of retention time of the user in activity information provided to the user;
(iii) one or more representatives of frequency of usage, e.g. indicative of an amount of activity information provided or caused providing;
(iv) or a combination thereof.

For instance, the one or more representatives of indicator information may be received by analyzing on which activity information provided to the user, the user has clicked on. In case the activity information is provided to the user by displaying the interest information by the terminal of the user, the user may for instance click on this information. This may for instance be indicative of the user being interested in the information provided to the user. Thus, the user signalizes interest in the provided interest information. The signalization information may for instance be part of and/or be stored as user behavior information, which may for instance be comprised by the user activity information.

Additionally or alternatively, one or more representatives of information being indicative of retention time of the user in activity information provided to the user may for instance be part of and/or be stored as user behavior information, which may for instance be comprised by the user activity information.

The user behavior information may for instance further be based on estimated or interpreted interest of the user in activity information provided to the user. For instance, in case a tracking results in the information that the activity information was provided to the user for a relatively long time compared to instantly aborting the provision of the activity information by the user after providing or causing providing the activity information, or skimming the provided or caused providing activity information by the user, it may be estimated or interpreted that the user is interested in the activity information.

According to an exemplary embodiment of all aspects, the user behavior information is determined by the usage of a weighting factor. The weighting factor may for instance be set by the user or be determined, e.g. by using the processor of the at least one apparatus, based on one or more inclusion and/or exclusion criterion, which may for instance be obtained respectively received by user input. For instance, the weighting factor can be relatively higher in case of e.g. a higher retention time associated to an activity information provided to the user compared to a lower retention time associated to an activity information provided to the user, which is provided to the user for a very short amount of time. For example, the provision of activity information to the user may be canceled in case the user clicks on another interest information provided to the user, or e.g. the user turns off the terminal on which the activity information provided to the user is canceled as well. Received user input (e.g. explicitly) stating the interest of the user in the activity information is weighted higher than the aforementioned estimated or interpreted interest of the user in the activity information.

According to an exemplary embodiment of all aspects, the determining of the adaptation of the activity information further comprises considering at least one profile information of the user (e.g. obtained in advance), wherein the profile information is indicative of at least one individual parameter associated with the user, e.g. a certain level (e.g. certain fitness level) the user intends to reach. Based on the at least one profile information a benchmarking characterizing the performance of the user may for instance be determined, e.g. by the processor of the at least one apparatus. The profile information may for instance comprise one or more interim objectives information, wherein the one or more interim objectives information may be indicative of a certain level (e.g. fitness level) of the user, wherein the certain level is below the level the user intends to reach. The interim objective information may for instance be used as the contextual information for motivating the user. The profile information may for instance be considered when determining the adaptation of the activity information. For instance, some activities may require a certain level in order to be performed by the user, e.g. due to requiring a certain strength, flexibility to name but a few fitness related examples.

Additionally or alternatively, the profile information may for instance be obtained based, at least in part, on one or more individual parameters associated with the user. The one or more individual parameters associated with the user may for instance be additionally indicative of predicted behavior of the user. For instance, one or more individual parameters may for instance be indicative of the frequency of usage of the present invention, e.g. an amount of times the method according to present invention is performed and/or controlled based on a request by the user. The frequency of usage of the present invention may for instance be indicative of a stage of behavior change of the user.

The behavior change of the user may for instance be divided into five stages. The behavior change may for instance be according to e.g. a transtheoretical model of behavior change. Alternatively or additionally, the behavior change may for instance be according to a user-defined model of behavior change. For instance, an exemplary model may be an integrative, biopsychosocial model to conceptualize the process of intentional behavior change. One or more stages of behavior change may for instance be defined, e.g. behavior change from precontemplation (e.g. the user not ready for a behavior change) to maintenance (e.g. the user has achieved a behavior change). The further stages of behavior change in between the two aforementioned from precontemplation to maintenance may for instance be contemplation (e.g. the user is getting ready for a behavior change), preparation (e.g. the user is ready for a behavior change) and action (e.g. the user takes action for a behavior change).

Based on the profile information indicative of a stage of behavior change of the user, different activity information may for instance be provided or caused providing, e.g. to the user. This may for instance support and enhance the behavior change of the user, for instance in order to reach a certain goal of the user (e.g. becoming healthier, more attractive, fitter, to name but a few examples).

In an exemplary embodiment according to all aspects, the profile information comprises at least one of the following individual parameters associated with the user:
(i) a representative of an age of the user;
(ii) a representative of a weight of the user;
(iii) a representative of a height of the user;
(iv) a representative of a body mass index of the user;
(v) a representative of a gender of the user;
(vi) a representative of a fitness level of the user;
(vii) a representative of one or more motivation aspects/motivators of the user;
(viii) a representative of one or more barriers of the user;
(ix) or a combination thereof.

The representative of an age of the user may for instance be an age of the user in years. The representative of a weight of the user may for instance be a weight of the user in kilograms (Kg). The representative of a height of the user may for instance be a height of the user in centimeters (Cm). The representative of a fitness level of the user may for instance be a value between a predefined range from a low value to a height value, e.g. 1 to 5.The representative of a motivation of the user may for instance be indicative of an attractiveness, performance, wellbeing, or a combination thereof of the user, which may for instance be a goal the user intends to reach by utilizing the present invention. The representative of one or more barriers of the user may for instance be indicative of a time, knowledge, motivation, health issue, or a combination thereof prevent the user from performing a certain activity of the activity information. Further, the representative of one or more barriers of the user may for instance be indicative of no barrier preventing the user from performing a certain activity of the activity information.

According to an exemplary embodiment of all aspects, the method further comprises: determining, e.g. by using the processor of the at least one apparatus, a fitness level information indicative of a current fitness level of the user based, at least in part, on the user activity information and/or on fitness information indicative of the current fitness level of the user obtained (e.g. received) by user input. Additionally or alternatively, the determining of the fitness level information may for instance be based on the first part of the user activity information, e.g. gathered by a sensor of an electronic device or terminal of the user.

According to an exemplary embodiment of all aspects, the method further comprising: determining one or more interim objectives information based, at least in part, on the profile information. For instance, intermediate objectives may be fulfilled by the user performing a certain respectively predetermined activity (e.g. exercise) or a certain respectively predetermined activity (e.g. exercise) comprised by a set of measures. Intermediate objectives may for instance be coupled to a grade of a certain difficulty, intensity, extension, pause, frequency of activity (e.g. exercise), which the user may need to fulfill in order to reach a certain level (e.g. certain fitness level). The determining of one or more interim objectives may be based on a current level (e.g. fitness level), e.g. obtained from the user activity information. Alternatively or additionally, the one or more interim objectives information may be determined by utilizing a look-up table comprising predefined one or more interim objectives information associated with a certain level (e.g. certain fitness level) a user intends to reach, e.g. comprised by the user activity information, and/or received by user input.

According to an exemplary embodiment of all aspects, the method further comprising: determining whether the user activity information has changed; and causing, at least in part, the determining of the adaptation of the activity information, e.g. provided to the user. The change of the user activity information may for instance be that the user activity information comprises new and/or additional information, e.g. with respect to (i) one or more inclusion criterion; (ii) one or more exclusion criterion; (iii) one or more controlling criterion; (iv) one or more influencing criterion; (v) one or more user behavior criterion; or a combination thereof.

It is to be understood that the presentation of the invention in this section is merely by way of examples and non-limiting.

Other features will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not drawn to scale and that they are merely intended to conceptually illustrate the structure and procedures described herein.

### BRIEF DESCRIPTION OF THE DRAWINGS

Having thus describing example embodiments in general terms, reference will now be made to the accompanying drawings, which are necessarily drawn to scale, and wherein
- Fig. 1: shows a schematic block diagram of a system according to an exemplary aspect of the present invention;
- Fig. 2: a flowchart showing an example embodiment of a method according to the first exemplary aspect of the present invention, for instance performed by server 200 of Fig. 1;
- Fig. 3: a flowchart showing an example embodiment, for instance performed by system 100 of Fig. 1; and
- Fig. 4: a schematic block diagram of an apparatus according to an exemplary aspect of the present invention.

### DETAILED DESCRIPTION

The following description serves to deepen the understanding of the present invention and shall be understood to complement and be read together with the description of the invention as provided in the above SUMMARY section of this specification.

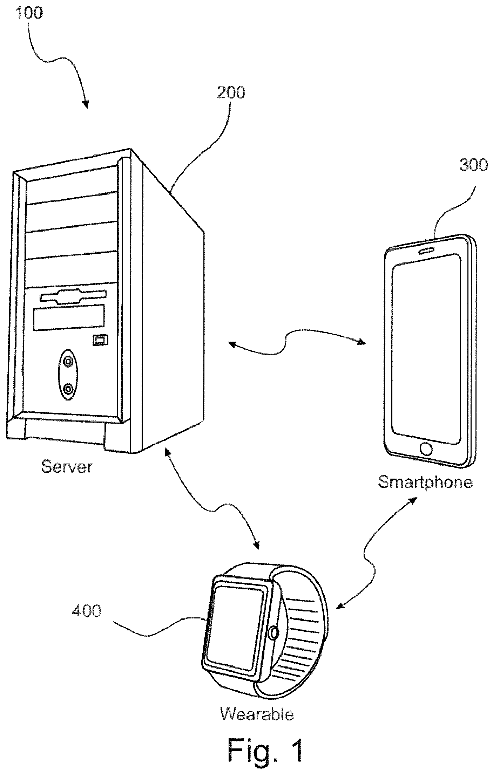

Fig. 1 is a schematic high-level block diagram of a system 100 according to an exemplary aspect. System 100 comprises a server 200, which may alternatively be embodied as a server cloud (e.g. a plurality of servers connected e.g. via the internet and providing services at least partially jointly), and a terminal or electronic device, of which a smartphone 300 and a wearable 400 as realizations are exemplarily shown.

According to embodiments, smartphone 300 obtains user activity information, and is provided with an activity information. Server 200 provides the activity information, and determines an adaptation of the activity information. Further, server 200 and/or smartphone 300 output the activity information. Wearable 400 may for instance be provided with the activity information as well.

Communications between server 200 and smartphone 300 and/or wearable 400 may for instance take place at least partially in a wireless fashion, e.g. based on cellular radio communication or on Wireless Local Area Network (WLAN) based communication, to name but a few examples. Further, communications between smartphone 300 and wearable 400 may for instance take place at least partially in a wireless fashion, e.g. Wireless Personal Area Network (WPAN), for instance Bluetooth, based communication, to name but an example. In this way, the mobility of the smartphone 300 and/or the wearable 400 is guaranteed.

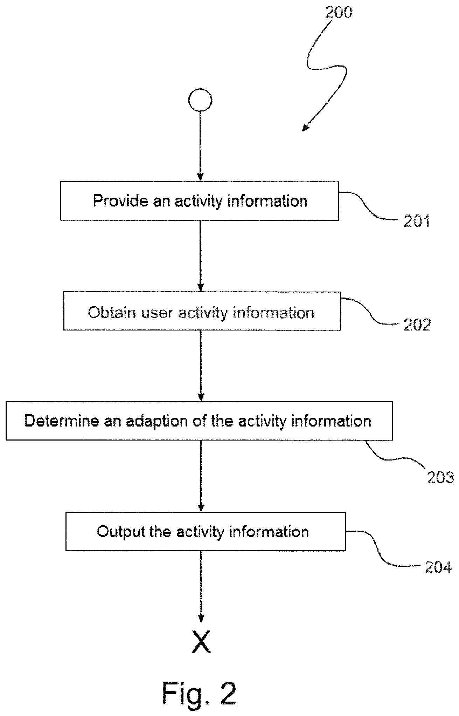

Fig. 2 is a flowchart 200 showing an example embodiment of a method according to the first exemplary aspect. The flowchart may for instance be performed by server 200 of Fig. 1.

In a first step 201, activity information is provided, e.g. from server 200 to smartphone 300 and/or to wearable 400. Further, activity information may be provided as well from smartphone 300 to wearable 400.

In step 202, user activity information is obtained (e.g. received), e.g. from smartphone 300 or from wearable 400 or from another entity. At least a first part of the user activity information is gathered by smartphone 300 or from wearable 400 or from another entity, e.g. by a sensor of the smartphone 300 or the wearable 400 or the other entity. At least a second part of the user activity information is received by user input.

In step 203, an adaptation of the activity information is determined based on the user activity information, e.g. by the server 200 of Fig. 1. This is done under the consideration of the respective user activity information. For instance, a received user input based on user behavior may for instance be indicative of a like or dislike of the activity information provided to the user. In case of a dislike, this user behavior is stored, e.g. in the user activity information. Thus, the user activity information has changed. For instance, the second part of the user activity information may for instance comprise a new exclusion criterion, e.g. obtained by user input (e.g. like or dislike of activity information provided, or an analysis of information obtained in conjunction with performing and/or controlling the present invention (e.g. movement information, profile information, or location information, to name but a few examples)). The determining of the adaptation of the activity information may for instance consider historical information of the user respectively historical user activity information. In this way, the adapted activity information may be more suitable for the current situation of the user. The same applies to a change of the user activity information caused by a change of the first part of the user activity information. This first part of the user activity information may for instance be received by information gathered by a sensor of smartphone 300 and/or wearable 400. For instance, the gathered information may be indicative of the activity (e.g. exercise) of the activity information provided being too hard or too easy for the user to be performed. Thus, the determining of the adaptation of the activity information may for instance consider an easier or more advanced activity (e.g. exercise) to be performed by the user and be comprised by the activity information, which may for instance be provided or caused providing in the next execution of the present invention.

The adapted activity information is outputted in a step 204, e.g. from the server 200 to the smartphone 300 or to the wearable 400 or to another entity that transmits the adapted activity information to a terminal or electronic device, e.g. smartphone 300 and/or wearable 400.

The exemplary flowchart 200 of Fig. 2 may for instance comprise one or more further features described above, for instance gathering movement information, e.g. with a sensor of smartphone 300 and/or a sensor of wearable 400, and determining a first and/or second feedback information, e.g. by server 200.

Furthermore, steps 202 and 203 may for instance be performed either serial, or in parallel respectively simultaneously.

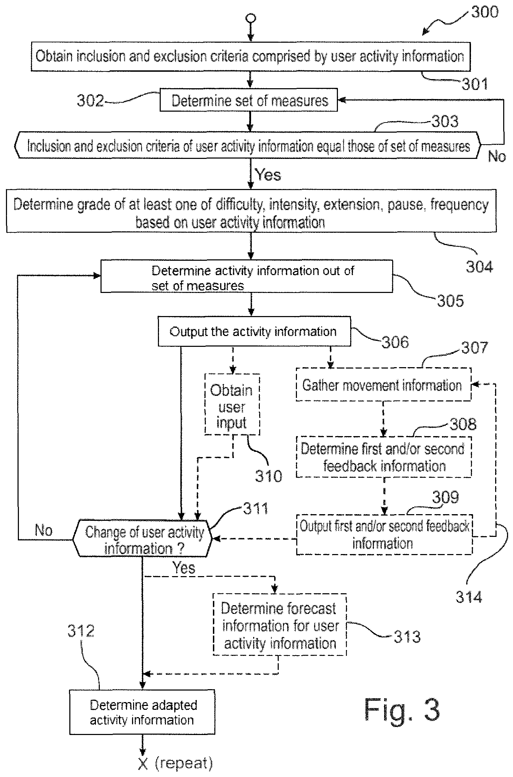

Fig. 3 is a flowchart showing an example embodiment for instance performed by system 100 of Fig. 1.

In step 301, inclusion and exclusion criteria comprised by user activity information are obtained (e.g. received). For instance, inclusion and exclusion criteria are received by user input, e.g. entered by a user using smartphone 300.

In step 302, a set of measures is determined, e.g. by server 200 of Fig. 1. A set of measures may for instance comprise one or more activity information.

In step 303, it is checked whether the obtained inclusion and exclusion criteria equal or nearly equal the inclusion and exclusion criteria associated to at least one of the activity information comprised by the set of measures. In case those criteria of the determined set of measures differ, e.g. by exceeding a predetermined threshold, the determined set of measures may not be suitable. Thus, another set of measures may be determined in step 302. In case the comparison has a positive result, it is proceeded with step 304.

In step 304, one or more grades with respect to difficulty, intensity, extension, pause, (trainings) frequency for an activity information comprised by the set of measures is determined. For instance, the set of measures may comprise an activity information representing the exercise 'lifting of pelvis'. Further, the set of measures may comprise the separate activity information representing 'Punching Bag Boxing' and 'Running'. Based on the obtained inclusion and exclusion criteria comprised by the user activity information, which may be received by user input, a certain metabolic equivalent of task (MET) factor may for instance be determined. The MET factor allows for instance to compare the energy consumption of certain activities. The MET factor may for instance be determined based on inclusion criteria of the user activity information, e.g. age, height, BMI, Gender, self associated fitness level of the user, motivational goal of the user, a certain barrier preventing the user to perform activity (e.g. exercise), or a combination thereof. Thus, the MET factor is indicative of a certain skill set respective level of the user, and may further be based on empirical values, e.g. one or more comparison values obtained by other users.

For instance, inclusion criteria may comprise a fitness level of 2 on a scale from 1 to 5, motivational goal is set to 'attractivity' and barrier is set to 'lack of motivation' and `lcnowledge'. Based e.g. on a predetermined rulebook, the difficulty can be determined as a difficulty of 2 on a scale from 1 to 5. Further, based on the barrier of 'lack of knowledge', a detailed instruction can be provided with respect to the exercise information representing a certain activity (e.g. exercise).

Further, based on the above mentioned inclusion criteria, the intensity of 2 on a scale from 1 to 5 can be determined, e.g. by the server 200. The activity information indicative of performing the activity (e.g. exercise) may be linked to an intensity of 2, which is associated with a duration of 5 to 10 seconds performing the activity (e.g. exercise).

The extension can be determined, e.g. by the server 200, in the way presented above, to 2 on a scale from 1 to 5 based on the inclusion criteria. Further, the activity information may be linked to the extension of 2, which is associated to 3 or 4 repetitions of the activity (e.g. exercise).

Based on the above mentioned inclusion criteria, the pause may be determined as being 2 on a scale from 1 to 5, e.g. by the server 200, which may be linked to a pause of 30 to 60 seconds between each repetition of the activity information indicative of an activity (e.g. exercise) to be performed by the user.

Further, based on the above mentioned inclusion criteria, the frequency of 2 on a scale from 1 to 5 may be determined, e.g. by the server 200. The frequency of 2 may be linked to a recommendation of a training frequency of up to two workouts per week.

Thus, a certain grade associated to difficulty, intensity, extension, pause, frequency may for instance be linked to certain parameters indicative of the kind of execution of the respective activity information.

In step 305, an activity information out of the determined set of measures is determined, e.g. by the server 200. In particular, the activity information is determined by considering the obtained inclusion and exclusion criteria. Further, real-time data, the current location of the user, a weather information or a season information, or a combination thereof may for instance be considered when determining the activity information. For instance, real-time data, the current location of the user, a weather information or a season information may be obtained (e.g. received) from a service of the internet, e.g. provided by server 200 of Fig. 1. To name an example, in case the season information is indicative of summer, and the weather is indicative of sunny weather, the activity information may be determined as representing 'Running', usually performed outdoors, instead of'Punching Bag Boxing', usually performed indoors.

In step 306, the activity information is outputted, e.g. from the server 200 to the smartphone 300 or to the wearable 400 of Fig. 1, or to another entity that transmits the adapted activity information to smartphone 300 and/or wearable 400 of Fig. 1.

Based on the outputted activity information, a user may perform the activity (e.g. exercise) represented by the activity information.

During this performance, in optional step 307 movement information may be gathered, e.g. by an accelerometer of the smartphone 300 and/or the wearable 400 of Fig. 1, or may be obtained by a tracking, e.g. performed and/or controlled by a terminal of the user. The tracking may for instance be a time-based tracking, e.g. an activity of an activity information takes a certain amount of time to be performed. Based on the gathered movement information, in optional step 308, a first and/or second feedback information may be determined. The first feedback information is indicative of whether the activity (e.g. exercise) associated to the activity information is performed correctly. The second feedback information is indicative of an effort of the user to perform the activity (e.g. exercise), e.g. evaluating whether the activity (e.g. exercise) was too hard, too easy or just right to be performed by the user.

In optional step 309, the first and/or second feedback information is outputted, e.g. to the smartphone 300 and/or wearable 400, or to another entity that transmits the first and/or second feedback information to smartphone 300 and/or wearable 400 of Fig. 1. Based on the first and/or second feedback information, at least one of an optical, an acoustical or a haptic feedback to the user may be provided to the user. Thus, the user may for instance be informed, e.g. in real-time, whether the performance of the activity (e.g. exercise) associated to the activity information needs improvement. Further, in case the performance of the activity (e.g. exercise) associated to the activity information may result in pain and/or in danger of injury for the user, the user can be provided feedback in order to prevent such risks.

In step 310, user input may for instance be obtained (e.g. received), e.g. user input entered into smartphone 300 or wearable 400 may be received by server 200 of Fig. 1. For instance, the user may like or dislike the provided activity information.

In step 311, it is determined whether a change of the user activity information has occurred, e.g. by the server 200. For instance, a change of user activity information may be caused by user input, e.g. obtained user input of step 310. Further, a change of user activity information may be caused by the first and/or second feedback information, e.g. by determining that the provided activity information has put the user at risk for pain and/or danger of injury.

In case no change of the user activity information has occurred, an alternative activity information out of the determined set of measures may be determined in step 305. Thus, the user can proceed with his training, e.g. in order to reach a certain fitness level or the like, which may be defined by a goal information.

In case a change of the user activity information has occurred, in step 312 an adapted activity information is determined, e.g. by the server 200. For instance, difficulty, intensity, extension, pause, frequency (or a combination thereof) may be adapted. Thus, the activity information is adapted in this way.

In optional step 313, a forecast information for the user activity information may be determined, e.g. by the server 200. For instance, in case user input of step 310 is obtained indicating that the user prefers outdoor exercises and/or activities, an adequate forecast information may be determined, which may for instance be considered when determining the adapted activity information in step 312.

The flowchart 300 may be repeated for providing a dynamically adaptive and interactively changing and individual user experience, e.g. by providing and/or outputting the adaptively determined activity information based on the user activity information.

The exemplary flowchart 300 of Fig. 3 may for instance be performed, at least partially by a server or server cloud, e.g. server 200 of Fig. 1. Apart from the dynamically adaptive and interactively changing and individual user experience, this enhances the energy consumption of the terminal and/or electronic device, e.g. smartphone 300, wearable 400. Despite using at least one mobile device - the terminal and/or electronic device - the energy consumption can be kept low enabling the method to accompany the user throughout his daily routines. Thus, minimal effort of the user is required for reaching e.g. a self-defined goal.

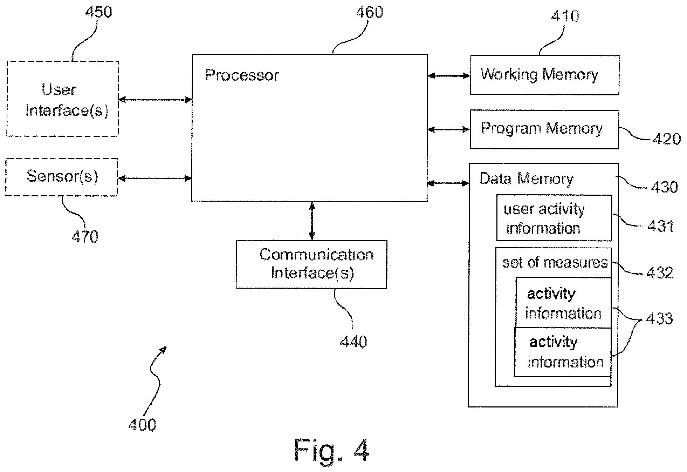

Fig. 4 is a schematic block diagram of an apparatus 400 according to an exemplary aspect of the present invention. Apparatus 400 may for instance represent server 200 of Fig. 1, or smartphone 300, or wearable 400 of Fig. 1, and may be configured to perform and/or control the flowchart 200 of Fig. 2.

Apparatus 400 comprises a working memory 410, program memory 420, data memory 430, communication interface(s) 440, optional user interface 450, processor 460, and optional sensor(s) 470.

Apparatus 400 may for instance be configured to perform and/or control or comprise respective means (410 to 470) for performing and/or controlling the method according to the first exemplary aspect. Apparatus 400 may as well constitute an apparatus comprising at least one processer (460) and at least one memory (420) including computer program code, the at least one memory and the computer program code configured to, with the at least one processor, cause an apparatus (e.g. apparatus 400) at least to perform and/or control the method according to the first exemplary aspect of the invention.

Processor 460 may for instance execute computer program code stored in program memory 420, which may for instance represent a computer readable storage medium comprising program code that, when executed by processor 460, causes processor 460 to perform the method according to the first exemplary aspect.

Processor 460 (and also any other processor mentioned in this specification) may be a processor of any suitable type. Processor 460 may comprise but is not limited to one or more microprocessor(s), one or more processor(s) with accompanying one or more digital signal processor(s), one or more processor(s) without accompanying digital signal processor(s), one or more special-purpose computer chips, one or more field-programmable gate array(s) (FPGA(s)), one or more controller(s), one or more application-specific integrated circuit(s) (ASIC(s)), or one or more computer(s). The relevant structure/hardware has been programmed in such a way to carry out the described function. Processor 460 may for instance be an application processor that runs an operating system.

Program memory 420 may also be included into processor 460. This memory may for instance be fixedly connected to processor 460, or be at least partially removable from processor 460, for instance in the form of a memory card or stick. Program memory 420 may for instance be non-volatile memory. It may for instance be a FLASH memory (or a part thereof), any of a ROM, PROM, EPROM and EEPROM memory (or a part thereof) or a hard disc (or a part thereof), to name but a few examples. Memory may also comprise an operating system for processor 460. Program memory 420 may also comprise a firmware for apparatus 400.

In the apparatus 400, further a working memory 410 is present, for instance in the form of a volatile memory. It may for instance be a Random Access Memory (RAM) or Dynamic RAM (DRAM), to name but a few non-limiting examples. It may for instance be used by processor 460 when executing an operating system and/or computer program.

Data memory 430 may for instance be non-volatile memory. It may for instance be a FLASH memory (or a part thereof), any of a ROM, PROM, EPROM, and EEPROM (or a part thereof) or a hard disc (or a part thereof), to name but a few examples. Data memory 430 may for instance store one or more activity information, as well as user activity information, as has already been described above. Furthermore, data memory 430 may store user activity information 431, and/or a set of measures 432. The set of measures 432 may for instance comprise one or more (or a plurality) of activity information.

Communication interface(s) 440 enable apparatus 400 to communicate with other entities, e.g. with smartphone 300, and/or wearable 400 of Fig. 1. The communication interface(s) 440 may for instance comprise a wireless interface (e.g. a cellular radio communication interface and/or a WLAN interface and/or a Bluetooth interface) and/or wire-bound interface, such as for instance an IP-based interface, for instance to communicate with entities via the internet.

User interface 450 is optional and may comprise a display for displaying information to a user and/or an input device (e.g. a keyboard, keypad, touchpad, mouse, etc.) for receiving respectively obtaining information from a user.

Sensor 470 is optional and may be used e.g. to gather movement information.

Some or all of the components of the apparatus 400 may for instance be connected via a bus. Some or all components of the apparatus 400 may for instance be combined into one or more modules.

The following embodiments shall also be considered to be disclosed:

### Embodiment 1:

An apparatus comprising at least one processor and at least one memory including computer program code; the at least one memory and the at least one computer program code configured to, with the at least one processor, cause the apparatus to at least perform:
- causing, at least in part, providing or providing an activity information indicative of an activity to be performed by a user;
- obtaining user activity information, wherein the user activity information is indicative of at least one inclusion criterion, at least one exclusion criterion, at least one controlling criterion, or a combination thereof, wherein at least a first part of the user activity information is gathered by a sensor and at least a second part of the user activity information is received by user input;
- determining, using a processor of the at least one apparatus, an adaptation of the activity information based on the user activity information;
- outputting the activity information.

### Embodiment 2:

The apparatus according to embodiment 1, the at least one memory and the computer program code configured to, with the at least one processor, cause the apparatus to perform:
- determining a contextual information based, at least in part, on the activity information, wherein the contextual information is indicative of further information associated with the activity information.

### Embodiment 3:

The apparatus according to embodiment 1 or embodiment 2, the at least one memory and the computer program code configured to, with the at least one processor, cause the apparatus to perform:
- obtaining movement information, wherein the gathered movement information is indicative of a movement caused by the activity performed by the user;
- determining a first feedback information indicative of whether the activity is performed correctly based on a comparison of the gathered movement information to at least one reference value information indicative of the activity being performed correctly;
- causing providing or providing the first feedback information.

### Embodiment 4:

The apparatus according to any of the embodiments 1 to 3, wherein the determining of the adaption is caused, at least in part, based at least on one or more of the following parameters:
(i) one or more movement information;
(ii) one or more location information representative to a current surrounding of the user and/or the position of the user;
(iii) one or more weather information representative to current weather conditions influencing an activity being possible to be performed;
(iv) one or more user input with respect to (a) at least one inclusion criterion; (b) at least one exclusion criterion; (c) at least one controlling criterion; (d) at least one influencing criterion; (e) at least one user behavior criterion; (f) or a combination thereof;
(v) or a combination thereof.

### Embodiment 5:

The apparatus according to any of the embodiments 1 to 4, wherein the determining of the adaptation is performed and/or controlled in predetermined time intervals.

### Embodiment 6:

The apparatus according to any of the embodiments 1 to 5, the at least one memory and the computer program code configured to, with the at least one processor, cause the apparatus to perform:
- determining a user behavior information based, at least in part, on user input, wherein the user behavior information is indicative of an interest of the user in the activity information provided to the user, wherein the user behavior information is determined based on at least one of the following:
   (i) one or more representatives of indicator information, wherein the indicator information is obtained by user input;
   (ii) one or more representatives of information being indicative of retention time of the user in activity information provided to the user.
   (iii) one or more representatives of frequency of usage frequency of usage;
   (iv) or combination thereof.

### Embodiment 7:

The apparatus according to embodiment 6, wherein the user behavior information is determined by the usage of a weighting factor.

### Embodiment 8:

The apparatus according to any of the embodiments 1 to 7, wherein determining of the adaptation of the activity information further comprises:
- considering at least one profile information of the user, wherein the profile information is indicative of at least one individual parameter associated with the user.

### Embodiment 9:

The apparatus according to any of the embodiments 1 to 8, wherein the profile information comprises at least one of the following individual parameters associated with the user::
(i) a representative of an age of the user;
(ii) a representative of a weight of the user;
(iii) a representative of a height of the user;
(iv) a representative of a body mass index of the user;
(v) a representative of a gender of the user;
(vi) a representative of a fitness level of the user;
(vii) a representative of one or more motivation aspects and/or motivators of the user;
(viii) a representative of one or more barriers of the user;
(ix) or a combination thereof.

### Embodiment 10:

The apparatus according to any of the embodiments 1 to 9, the at least one memory and the computer program code configured to, with the at least one processor, cause the apparatus to perform:
- determining one or more interim objectives information based, at least in part, on the profile information.

### Embodiment 11:

The apparatus according to any of the embodiments 1 to 10, the at least one memory and the computer program code configured to, with the at least one processor, cause the apparatus to perform:
- determining whether the user activity information has changed;
- causing, at least in part, the determining of the adaptation of the activity information provided to the user.

In the present specification, any presented connection in the described embodiments is to be understood in a way that the involved components are operationally coupled. Thus, the connections can be direct or indirect with any number or combination of intervening elements, and there may be merely a functional relationship between the components.

Moreover, any of the methods, processes and actions described or illustrated herein may be implemented using executable instructions in a general-purpose or special-purpose processor and stored on a computer-readable storage medium (e.g. disk, memory, or the like) to be executed by such a processor. References to a 'computer-readable storage medium' should be understood to encompass specialized circuits such as FPGAs, ASICs, signal processing devices, and other devices.

The expression 'A and/or B' is considered to comprise any one of the following three scenarios: (i) A, (ii) B, (iii) A and B. Furthermore, the article 'a' is not to be understood as 'one', i.e. of the expression 'an element' does not preclude that also further elements are present. The term 'comprising' is to be understood in an open sense, i.e. in a way that an object that 'comprises an element A' may also comprise further elements in addition to element A.

It will be understood that all presented embodiments are only exemplary, and that any feature presented for a particular example embodiment may be used with any aspect of the invention on its own or in combination with any feature presented for the same or another particular example embodiment and/or in combination with any other feature not mentioned. In particular, the example embodiments presented in this specification shall also be understood to be disclosed in all possible combinations with each other, as far as it is technically reasonable and the example embodiments are not alternatives with respect to each other. It will further be understood that any feature presented for an example embodiment in a particular category (method/apparatus/ computer program/system) may also be used in a corresponding manner in an example embodiment of any other category. It should also be understood that presence of a feature in the presented example embodiments shall not necessarily mean that this feature forms an essential feature of the invention and cannot be omitted or substituted.

The sequence of all method steps presented above is not mandatory, also alternative sequences may be possible. Nevertheless, the specific sequence of method steps exemplarily shown in the figures shall be considered as one possible sequence of method steps for the respective embodiment described by the respective figure.

The invention has been described above by means of example embodiments. It should be noted that there are alternative ways and variations that are obvious to a skilled person in the art and can be implemented without deviating from the scope of the appended claims.

## Claims

1. A method, performed by at least one apparatus, comprising:
- causing, at least in part, providing or providing an activity information indicative of an activity to be performed by a user;
- obtaining user activity information, wherein the user activity information is indicative of at least one inclusion criterion, at least one exclusion criterion, at least one controlling criterion, or a combination thereof, wherein at least a first part of the user activity information is gathered by a sensor and at least a second part of the user activity information is received by user input;
- determining, using a processor of the at least one apparatus, an adaptation of the activity information based on the user activity information;
- outputting the activity information.

2. The method of claim 1, further comprising:
- determining a contextual information based, at least in part, on the activity information, wherein the contextual information is indicative of further information associated with the activity information.

3. The method of claim 1 or claim 2, further comprising:
- obtaining movement information, wherein the gathered movement information is indicative of a movement caused by the activity performed by the user;
- determining a first feedback information indicative of whether the activity is performed correctly based on a comparison of the gathered movement information to at least one reference value information indicative of the activity being performed correctly;
- causing providing or providing the first feedback information.

4. The method of any of the preceding claims, wherein the determining of the adaption is caused, at least in part, based at least on one or more of the following parameters:
(i) one or more movement information;
(ii) one or more location information representative to a current surrounding of the user and/or the position of the user;
(iii) one or more weather information representative to current weather conditions influencing an activity being possible to be performed;
(iv) one or more user input with respect to (a) at least one inclusion criterion; (b) at least one exclusion criterion; (c) at least one controlling criterion; (d) at least one influencing criterion; (e) at least one user behavior criterion; (f) or a combination thereof;
(v) or a combination thereof.

5. The method of any of the preceding claims, wherein the determining of the adaptation is performed and/or controlled in predetermined time intervals.

6. The method of any of the preceding claims, further comprising:
- determining a user behavior information based, at least in part, on user input, wherein the user behavior information is indicative of an interest of the user in the activity information provided to the user, wherein the user behavior information is determined based on at least one of the following:
(i) one or more representatives of indicator information, wherein the indicator information is obtained by user input;
(ii) one or more representatives of information being indicative of retention time of the user in activity information provided to the user.
(iii) one or more representatives of frequency of usage;
(iv) or combination thereof.

7. The method of claim 6, wherein the user behavior information is determined by the usage of a weighting factor.

8. The method of any of the preceding claims, wherein determining of the adaptation of the activity information further comprises:
- considering at least one profile information of the user, wherein the profile information is indicative of at least one individual parameter associated with the user.

9. The method of claim 8, wherein the profile information comprises at least one of the following individual parameters associated with the user::
(i) a representative of an age of the user;
(ii) a representative of a weight of the user;
(iii) a representative of a height of the user;
(iv) a representative of a body mass index of the user;
(v) a representative of a gender of the user;
(vi) a representative of a fitness level of the user;
(vii) a representative of one or more motivation aspects and/or motivators of the user;
(viii) a representative of one or more barriers of the user;
(ix) or a combination thereof.

10. The method of claim 8 or claim 9, further comprising:
- determining one or more interim objectives information based, at least in part, on the profile information.

11. The method of any of the preceding claims, further comprising:
- determining whether the user activity information has changed;
- causing, at least in part, the determining of the adaptation of the activity information provided to the user.

12. An apparatus comprising
at least one processor and at least one memory including computer program code, wherein the memory and computer program code are configured to, with the processor, cause the apparatus to perform and/or control a method according to one of the claims 1 to 11; or comprising means for performing and/or controlling a method according to one of the claims 1 to 11.

13. System, comprising:
- one or more apparatuses, in particular at least one apparatus and a terminal, which are adapted to at least perform and/or control a method according to any of the preceding method claims.

14. Computer program code configured, upon execution by a processor, to cause an apparatus to perform and/or control a method according to one of the claims 1 to 11.
